# EUROPEAN PATENT SPECIFICATION

(11) **EP 2 329 794 B1**
(45) Date of publication and mention of the grant of the patent: **23.07.2014**
(21) Application number: 10394025.0
(22) Date of filing: 06.12.2010
(51) Int. Cl.: A61F 2/01, A61M 29/02

(54) **A perfusion device**
Perfusionsvorrichtung
Dispositif de perfusion

(30) Priority: 07.12.2009 IE 20090916
(43) Date of publication of application: 08.06.2011
(73) Proprietor: University of Limerick, Limerick (IE)
(72) Inventor: Walsh, Michael, Glin, County Limerick (IE); O'Connell, Barry, Raheen, Limerick (IE); O'Donnell, Michael, Tipperary Town, County Tipperary (IE); McGloughlin, Timothy, Ballyclough, County Limerick (IE); Lawlor, Michael, Corbally, Limerick (IE)
(74) Representative: Weldon, Michael James

(56) References cited:
- WO-A1-00/12169
- US-B1- 6 254 563

## Description

### Field of the Invention

The invention relates to medical devices, and more particularly to devices for insertion in blood vessels and having a perfusion balloon for expanding to press against the vessel wall.

### Prior Art Discussion

The balloon catheter is well established as an important medical device. The device can be used on its own to open a blocked artery or it can be used to deliver a stent (a small wire mesh tube) to the offending artery. The stent will expand when the balloon expands and is left in the artery acting as a scaffold to keep it open.

Carotid angioplasty and stenting is being used widely to treat severe carotid obstructive disease. An acute complication of carotid angioplasty and stenting relates to the distal embolisation of plaque particles during the endovascular procedure, with the risk of definitive stroke. Studies using *in vivo* monitoring (such as Magnetic Resonance Imaging (MRI), Computed Tomography (CT) or Transcranial Doppler) have shown that carotid stenting is associated with a higher incidence of transient ischaemic attack (TIA) or stroke from embolisation of plaque fragments in comparison to surgical endarterectomy. To reduce the possibility of these plaque fragments causing periprocedural neurological complications, cerebral protection is used. Embolic protection has also been used in other arteries, such as coronary and renal arteries, and therefore the technology is not limited to arteries belonging to the carotid bifurcation.

EP1400257 (Embol-X Inc) describes a percutaneous stent deployment assembly comprising a balloon and a filter for capturing emboli. It includes a conical-shaped filter which extends across the full cross-sectional area of the vessel. US5545135 describes a perfusion balloon stent comprising a balloon which is annular in cross-section enabling blood flow through its central passageway.

WO 0012169 discloses a filter extending distally of the balloon filtering the blood vessel cross section.

The invention is directed towards providing an improved perfusion device for applications such as treatment of blocked blood vessels, with reduced disruption of blood flow during the surgical procedure and/or with limiting of the host tissue response which would reduce flow due to perceived high blood pressure.

### Summary of the Invention

According to the invention, there is provided a vascular device as set out in claim 1.

The filtering portion is configured to, when deployed, extend in a direction which is at least partly radial, and then in a direction which is at least partly proximal.

In one embodiment, the filtering portion is substantially frusto-conical in shape when deployed.

In one embodiment, the filter has a proximal stem which is attached to the perfusion balloon or a support for the perfusion balloon.

In one embodiment, the filtering portion has an asymmetrical edge for improved retrieval to enable improved device deliverability and extraction.

In one embodiment, the filter comprises a retainer adapted to retain an end of the filtering portion in an in-use position abutting a vessel wall.

In one embodiment, the retainer comprises members extending from the perfusion balloon or a support for the perfusion balloon.

In one embodiment, the members include wires.

In one embodiment, the retainer is adapted to apply a radially outward bias to urge the filter to abut a vessel wall.

In one embodiment, the retainer comprises cantilevered members.

In one embodiment, the retainer is ring-shaped having a configuration conforming to a vessel perimeter.

In one embodiment, the retainer is inflatable.

In one embodiment, the retainer is linked with the balloon for simultaneous inflation.

In one embodiment, the device further comprises an internal support for the perfusion balloon.

In one embodiment, the support is adapted to allow gradual and controlled inflation of the perfusion balloon.

In one embodiment, the support comprises a scaffold structure.

In one embodiment, the scaffold structure comprises elements of a shape memory material.

In one embodiment, the support comprises at least one element in the form of opposed arches.

In one embodiment, the support comprises an element in the form of a coil.

In another embodiment, the support comprises an inner balloon adapted to fit within the perfusion balloon.

In one embodiment, the inner and perfusion balloons are coiled, the coil pitches being in or out of phase.

In one embodiment, the device is adapted to support a stent around the perfusion balloon and a mechanism for delivery of the stent by inflation of the perfusion balloon.

In another aspect, it is disclosed a method of performing a procedure using a vascular device comprising a perfusion balloon having, when inflated, a through-hole for blood flow, and a distal filter for trapping emboli, the filter being configured to filter an annular cross-sectional area extending from a vessel wall while leaving an un-filtered central passageway, the method comprising the steps of:
inserting the device into a vessel,
inflating the perfusion balloon while allowing blood to flow through the through-hole, the filter capturing emboli which are dislodged from the vessel wall while allowing unimpeded blood flow through the central passageway

In one embodiment, the filter is supported by retainers to engage the vessel wall.

In one embodiment, the retainer is inflatable and is inflated together with the perfusion balloon so that it engages around the vessel wall.

### Detailed Description of the Invention

The invention will be more clearly understood from the following description of some embodiments thereof, given by way of example only with reference to the accompanying drawings in which:-
Fig. 1 is a cut-away perspective view of a perfusion device of the invention when deployed, the device having an annular balloon and a support rib cage balloon and an embolic protection filter;
Fig. 2 is a partly cut-away perspective view showing an alternative filter, having a wall apposition support mechanism using an inflated ring balloon;
Fig. 3 is a cut-away perspective view of an alternative device, in this case having a metallic support coil;
Fig. 4 is a partly cut away view showing part of an alternative device, in this case having a mechanical support with arched metal members when fully deployed;
Fig. 5 is a longitudinal cross-sectional view showing site of occlusion of plaque B within an artery wall A;
Fig. 6 is a longitudinal cross-sectional diagram showing positioning of the device of Fig. 3; and Fig. 7 is a similar view showing deployment of the device in which a catheter sheath is retracted thus deploying the filter support wires and the filter mesh; Figs. 8 and 9 show inflation of the device in which the support inhibits collapse of the inner surface of the perfusion balloon and supports the outward inflation of the balloon and hence deformation of the occlusion and release of emboli which are captured by the filter; and Fig. 10 shows retrieval of the device;
Fig. 11 is a diagrammatic cross-sectional view showing the device of Fig. 1 when deployed, in which operation of the rib cage balloon is illustrated;
Fig. 12 is a set of three views showing a filter for a device of various embodiments and a cross-sectional view of an alternative filter, these views illustrating that the filter screens emboli only along an annular cross section with remaining blood being allowed to perfuse uninhibited through the lumen of the filter;
Fig. 13 is a plot showing competing physical phenomena due to the presence of a support scaffold, in which as the inner lumen diameter increases to relieve the fluid flow pressure drop across the device, the associated strain applied to the occluding plaque also increases due to increasing diameter;
Fig. 14 is a plot showing stress and strain uniaxial characterisation tests of fresh human carotid plaque with varying plaque type and stiffness;
Fig. 15 is a plot showing strain at the inner and outer diameters of an internal carotid artery (ICA) associated with dilation of a 1 and 2 step dilation balloon;
Fig. 16 is a plot showing velocity profiles along the centre of a vessel for three device configurations, in which a filter region is highlighted within a box and the direction of blood flow is noted with an arrow, and Fig. 17 shows velocity contours and direction for the three device configurations of Fig. 16;
Fig. 18 is a cut-away cross-sectional view showing a part of another embodiment of filter for devices of the invention, the filter having an alternative filter wall apposition support mechanism;
Fig. 19 is a cut-away cross-sectional view showing a part of a device of another embodiment, having an inner rib cage support balloon and an outer coiled balloon;
Fig. 20 is a cut-away cross-sectional view showing a part of a device of another embodiment, having an inner coiled support balloon and an outer coiled perfusion balloon;
Fig. 21 is a cut-away cross-sectional view showing a device of another embodiment of the invention, demonstrating that an inner coiled support balloon and an outer coiled perfusion balloon can have different pitch angles and orientation in relation to one another; and
Figs. 22 and 23 are cut-away cross-sectional views showing parts of alternative devices having inner and outer coiled support and perfusion balloons.

### Description of the Embodiments

### Overview

Devices of the invention in various embodiments include an annular embolic filter for embolic protection device combined with a perfusion balloon. When the balloon is deployed, the filter allows for emboli-free blood to flow through the region of treatment. Blood is allowed to pass uninhibited during the treatment, while at the same time the filter protects the cranial vascular system from emboli dislodged from the artery wall upon inflation of the perfusion balloon. This results in a minimum loss in blood pressure during treatment, in contrast to many prior devices having filters which extend across the full cross section. Also, the latter devices may result in losses in blood pressure to vessels distal of the treatment site as the filter captures and becomes saturated with emboli. Thus, by providing, in a counter intuitive manner, a filter with a hole in the middle of the device, optimum emboli filtration can be achieved and loss of blood pressure can be minimised during deployment.

### Description with reference to the drawings

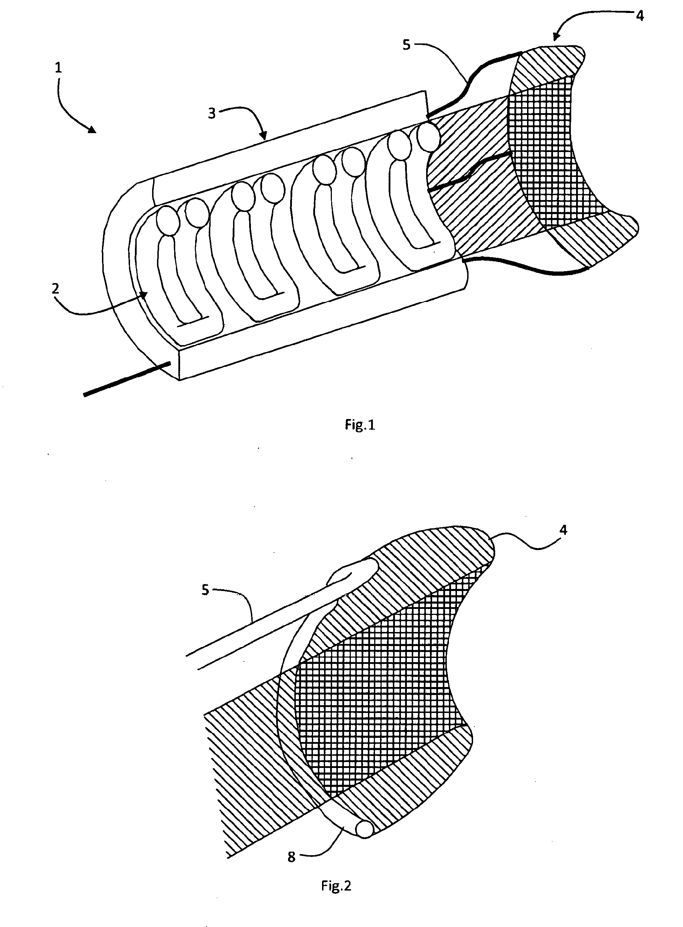

Referring to Fig. 1 a perfusion device 1 of the invention comprises an inner support structure 2, an outer inflatable balloon 3, and an embolic protection filter 4. The support structure 2 is a balloon having a rib-cage configuration, and the outer balloon 3 has an elongated annular configuration. The filter 4 has a membrane which extends along the length of both balloons, providing stability for the balloons and also offering further protection during the balloon/stent deployment of the device. Importantly, the filter 4 covers only an annular cross-sectional region, having a central passageway to allow emboli-free blood to flow and being folded back on itself adjacent the vessel wall creating a basket type effect and held in place by stays 5. The latter are of a metal which is biased radially outwardly to retain the filter mesh open. The annular filter 4 extends from the distal end of the device, and is retained in its annular shape by the support wires 5, which are of a shape memory alloy, extending from the distal end of the balloon.
Fig 2 shows an alternative filter support 8, namely a ring balloon structure inflated to maintain artery wall contact during the procedure. This 'filter ring' can have standalone inflation or inflation can be initiated by inflation of the inner balloon 2 and will provide full lumen wall apposition.

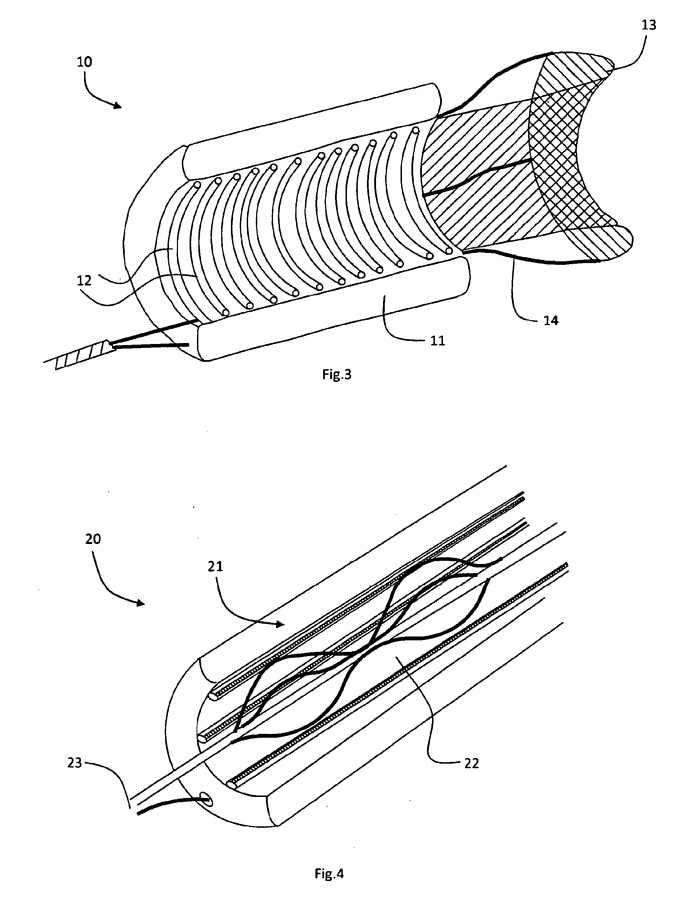

Fig. 3 illustrates a device 10 of an alternative embodiment. There is an elongate outer annular balloon 11 and an inner support structure 12. The inner support structure 12 consists of a shape memory support coil, which is not attached to the outer balloon 11. The inner support coil 12 acts as support to the outer annular balloon 11 to prevent collapse of the inner diameter of the outer balloon 11.
Fig. 4 illustrates a perspective cut-away view of a device 20 of an alternative embodiment. There is an elongate outer annular balloon 21, and a mechanical inner support 22 on a catheter shaft 23. The inner support structure 22 consists of a radial array of support arches, made from a shape memory alloy that can be distended when required to support the outer annular balloon 21, and retracted when not in use.

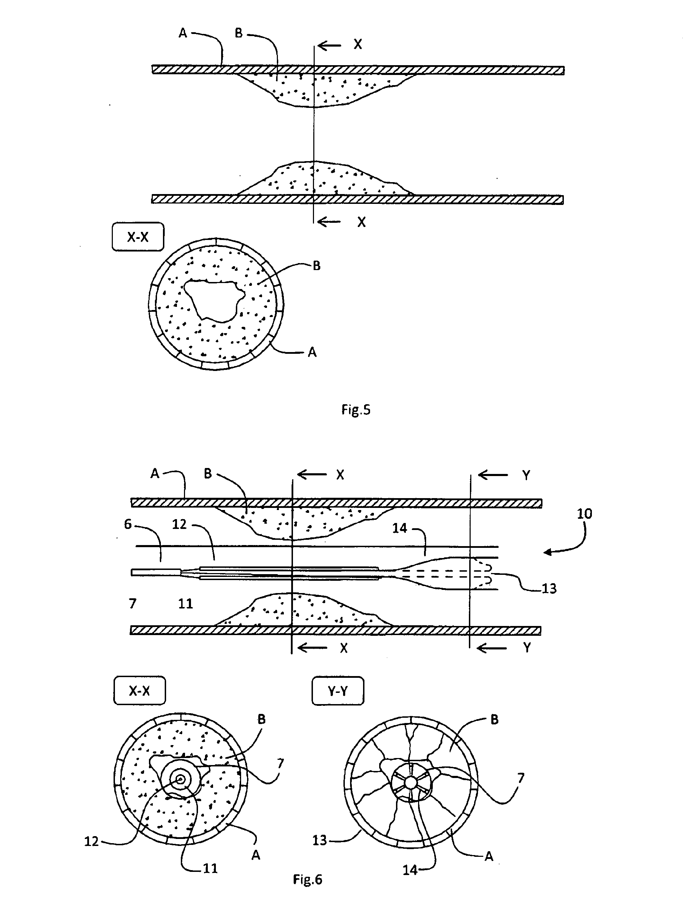

Figs. 5 to 10 are longitudinal and transverse cross-sectional views showing deployment and removal of the device of Fig. 3 in an occluded artery.

### Stage 1

Fig. 5 shows an occluded arterial vessel A. Prior to deployment of the device 10, a guide-wire must navigate through the arterial network and traverse this occlusion to facilitate the safe deployment of the balloon and filter.

### Stage 2

Once the guide wire is in place, the device 10 with the perfusion balloon 11 is positioned within the site of the occlusion B and the filter 13 distal of the occlusion site, Fig. 6.

### Stage 3

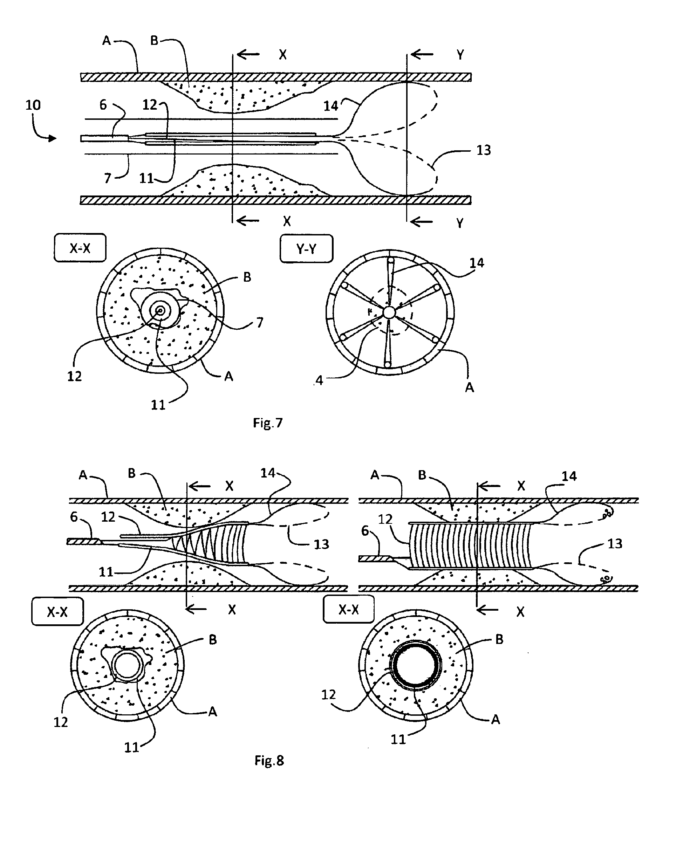

Once the device is in place, the catheter sheath 7 is retracted. As the catheter sheath 7 is removed, the filter 13 is deployed, Fig. 7. It is envisaged that the filter support wires 14 are of a shape memory alloy, so that when the catheter sheath 7 is removed, the filter wires 14 deform to their predefined shape and orientation. An alternative mechanism of filter wall contact can also be used. The number of filter support wires can vary and can be equally spaced in the circumferential direction, Fig. 7 - cross-section Y-Y. The maximum number of support wires 14 is limited to the trackability and stiffness of the device within the catheter. This encapsulates the site of the occlusion and thus captures any emboli released during the angioplasty procedure.

### Stage 4

Once the catheter sheath 7 is removed, the inner support can be deployed, Fig. 8. The support coil 12 of the device 10 is of a shape memory alloy and prior to deployment, the coil 12 is in a stretched configuration within the catheter. As the coil is unwrapped the coil takes to its predefined shape. The coil 12 acts as an initial angioplasty device, deforming the offending plaque/disease/occlusion B prior to inflation of the outer balloon 11. As the plaque/disease/occlusion B deforms at this stage, the perfusion filter device is in place to capture released emboli. The deployment of the support coil 12 immediately permits the flow of blood beyond the catheter and retains it for the entire procedure. If an alternative balloon support of the device, as per Figs. 1, 19, 20, and 21, were to be used it would also be deployed at this stage, prior to the inflation of the outer balloon.

### Stage 5

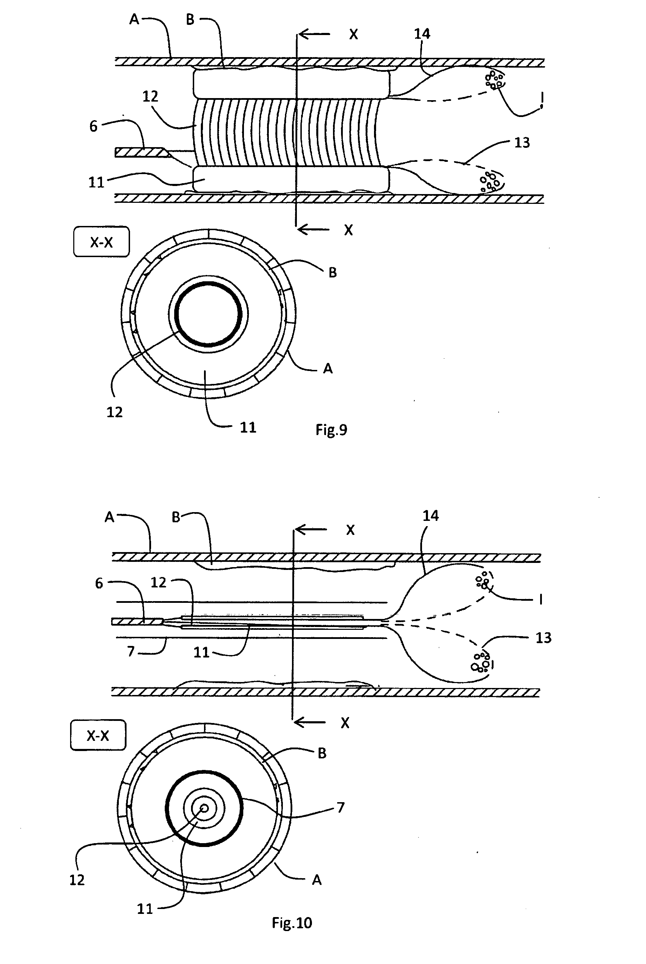

Once the support coil 12 is deployed the balloon 11 can be inflated, Fig. 9. Gradual inflation of the outer balloon 11 is possible due to the support coil, or other support mechanism, retaining a lumen for continuous antegrade blood supply. The process of controlled inflation is important as current angioplasty techniques using standard balloons result in more frequent persistent hypotension in patients. This is due to the fact that during current methodologies of these procedures, the balloon angioplasty devices over-stretch the arterial wall/plaque/diseased artery, inducing para-sympathetic signals from the host tissue, reducing the work rate of the heart and increasing hypotension of the patient. Overstretching of the vessels is a default of current carotid angioplasty procedures due to the small window of time for cranial blood flow to be stopped. Gradual inflation of the perfusion balloon allows time for the host tissue to adjust to these pressures and stretches, and also reduces the potential for recoil of the arterial vessel once the perfusion balloon is removed.

### Stage 6

After a desired inflation period the device 10 can be removed. The perfusion balloon 11 is deflated and the support coil 12 is retracted. The catheter retrieval sheath 7 is deployed to encapsulate the perfusion balloon 11. The device remains deployed for a period of time after the collapse of the perfusion balloon 11 as to capture any emboli dislodged during the angioplasty procedure. The filter 13 containing any plaque is then collapsed by further deploying the catheter retrieval sheath 7. Once the catheter retrieval sheath 7 fully encapsulates the embolic protection system, the entire device is removed, leaving a patent artery in its wake.

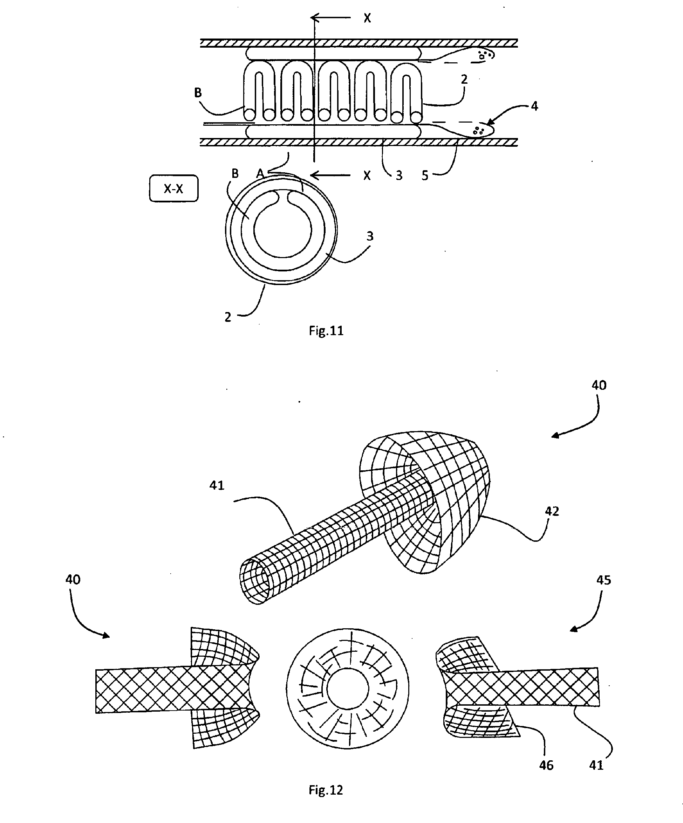

Fig. 11 illustrates the full deployment of the device 1 of Fig. 1 with the rib cage balloon support 2.

Fig. 12 illustrates an annular embolic protection filters 40 and 45 for use in various embodiments. In the geometrical design of the filter 40 the outer diameter is normal to the lumen surface, and in the filter 45 the outer diameter of the filter basket is asymmetrical, at an angle to the surface of the artery wall, creating an elliptical edge, which may improve device functionality in a number of areas. The filter 45 creates an elliptical contact with the artery wall due to the development/formation of a contact slope/angle on the filter basket. This basket angle may be used to aid in device crimping prior to deployment, to improve stability and wall apposition within the artery due to an increase in artery wall contact, and to improve deliverability and ease of withdrawal into the catheter sheath. This filter basket angulation is achieved in the manufacturing/production of the device and is not as a result of its deployment.

The preferred filter materials are including but limited to; compliant polymers such as polyurethane, Latex, Silicone, polyethylene, polyethylene terephthalate (PET), nylon, or shape memory materials such as superelastic nickel titanium alloy, or stainless steel. The filter material may also include an anti-thrombogenic coating. A second material is also envisaged containing a metallic element which can be visualised within the blood vessel using fluoroscopic imaging or angiography imaging, which will act as a locator to the operator.

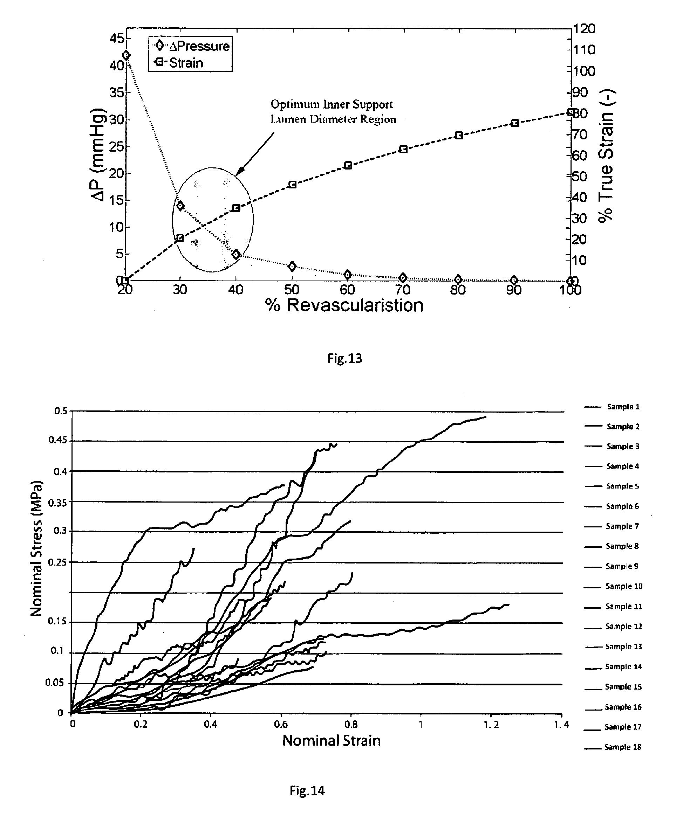

Fig. 13 to 17 demonstrate the performance of various aspects of the device and also the behaviour of the arterial plaques within the arteries.

The design criterion for the support scaffold is a function of initial strain applied to the occluding plaque, flow rate and pressure drop across the length of the device. As a result, the support scaffold is designed to minimise the impacts of these physical phenomena on the performance of this device. A large inner support lumen diameter is required to minimise the pressure drop (ΔP) across the device. However, as the lumen diameter increases, the result is an increasing strain on the occluding plaque, Fig. 13. Uni-axial tensile experiments carried out to characterise arterial plaque have shown unique rupture strains exist for plaques of varying stiffness, Fig 14. Fig. 13 illustrates the mechanics of the physical phenomena employed to define the geometry of the support scaffold. The highlighted region illustrates the region where these competing physical phenomena are at a minimum, thus maximising the effectiveness of the support scaffold. The stiffness of hard type plaque is employed in determining the inner support diameter as soft plaque type is capable of withstanding much greater strains, before rupture.

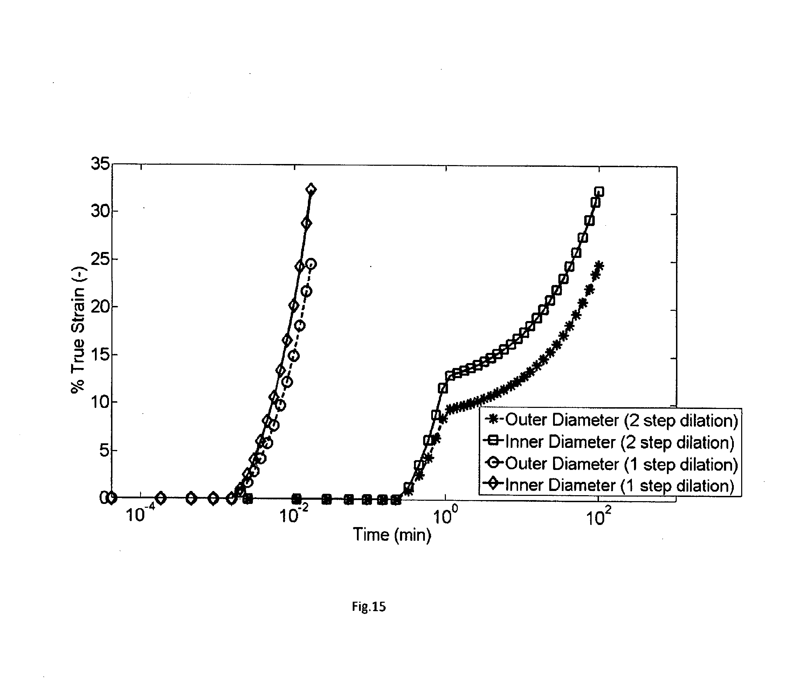

Hemodynamic instabilities during CAS have been ascribed to the consequences of direct carotid host tissue stimulation during balloon inflation [1], [2], [3], and can be described through the presence of hypotension, bradycardia and/or asystole. Mangin *et al.* (2003) found that CAS stimulates host tissue in all patients studied, resulting with a markedly decrease in heart rate and blood pressure. Gupta *et al.* (2005) reports that hemodynamic instability after CAS occurs in 29% to 51% of patients undergoing the procedure. Dangas *et al.* (2000) found that of 140 patients who underwent CAS, those with hypotension were more likely to suffer a minor stroke after the procedure [4]. Abou-Chebl *et al.* (2004) reports that from a total of 404, patients with haemodynamic instabilities had a significantly increased risk of stroke (odd ratio (OR) = 2.6, 95% CI 1.2-5.9), myocardial infarction (OR=4.5, 95% CI 1.2-16.9) or death (OR=2.7, 95% CI 1.0-7.6) in the peri-operative period. The odds ratio for the combined endpoint of stroke, myocardial infarction or death was 3.6 (95% CI 1.8-6.9) in patients with haemodynamic instability. Reasons for hypotensive related stroke have been related to low flow and low blood pressure resulting from damage to the host tissue prior to CAS (during atherosclerotic build-up) or peri-procedural over-extension during CAS, (Gupta *et al.,* 2005). The novelty of a two step dilation of occluded arterial vessel allows the reduction of overstretching of the host tissue, located in the carotid sinus. The first dilation step is instantaneous (similar to traditional current angioplasty balloon devices) but with small strains applied to the plaque preventing rupture, with a prolonged second dilation step, Fig 15. For Fig. 15, the second dilation time step of the novel device is for example 4 magnitudes longer than that of traditional/current devices. This controlled second dilation step can be used with continuous monitoring of heart rate and blood pressure to minimise peri- and post- operative haemodynamic instabilities and other adverse events.

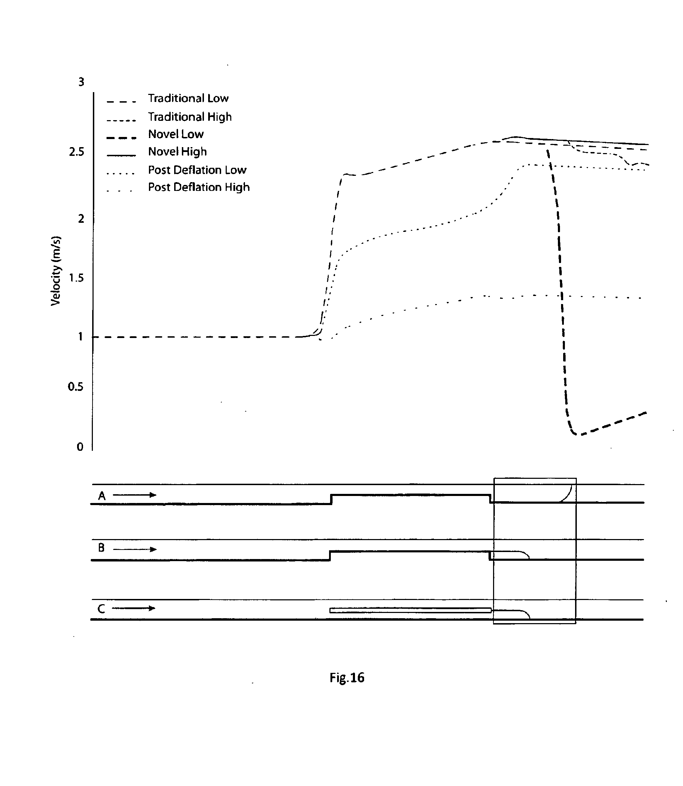

Fig. 16 demonstrates the velocity profiles along the centre of the artery as the blood passes from a full 5mm vessel through the perfusion balloon and finally through a filter. Model A shows the perfusion balloon used in conjunction with a traditional catheter, model B shows the complete device with perfusion filter and model C shows the complete device upon deflation of the outer balloon before device retrieval. Each filters configuration was modelled as having high permeability, i.e. not blocked by emboli, and low permeability demonstrating the effect of a filter after capturing a sufficient amount of plaque. As filter A becomes clotted with emboli the velocity beyond the filter reduces dramatically. When deployed, the proposed device, model B, doesn't have this draw back because the filter enables the majority of blood flow through the device because it is emboli free. The clotting of the filter will affect the device once the outer balloon is deflated, model C, but as the filter becomes less permeable, the blood flow redirects itself through the perfusion balloon and an increase in blood velocity is seen, which is in stark contrast to model A.

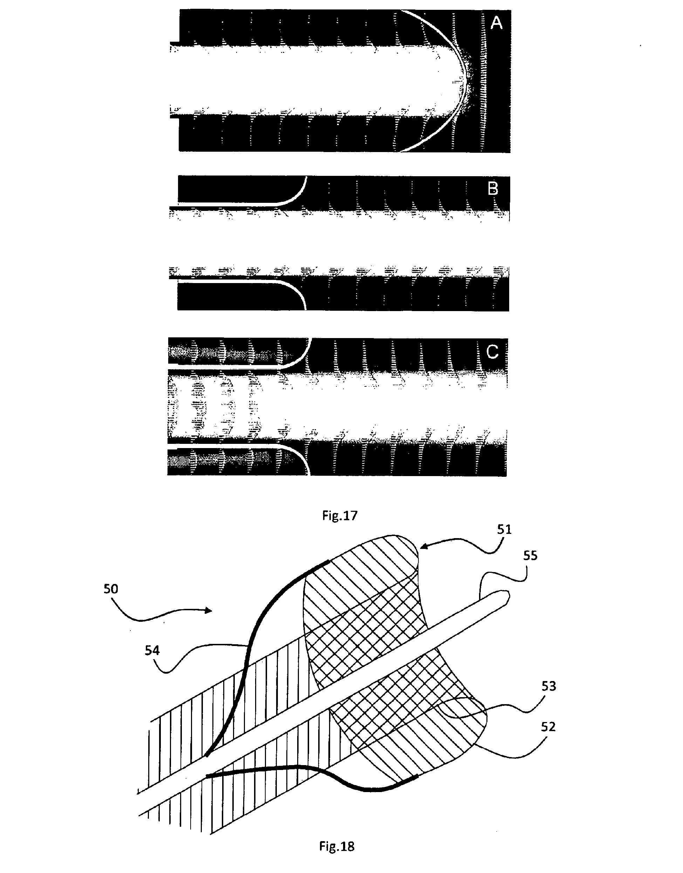

Fig. 17 takes a closer look at the velocity contours and directions surrounding the filters that have been clotted with emboli for the 3 device configurations, highlighted in the box in Fig. 16. The restriction of blood flow is apparent in models A and C. However, model C permits the flow of emboli free blood through the device even if the filter becomes completely blocked. Therefore the device addresses both of the key design criteria associated with embolic devices, the filter can have a small pore size, so as to catch all required emboli, and it will enable continuous blood flow through the device to the brain. After traditional carotid angioplasty the embolic protection device remains within the vessel to capture post deflation plaque fragments that become disassociated from the wall. The issue surrounding this is the creation recirculating blood around the filter which increases blood residency times and can lead to thrombus formation.

The invention hence avoids the prior art problem of a catheter creating a blockage upon inflation. Not only can this prior approach result in an ischemic attack but it severely restricts the time that the physician has to propagate the artery. The invention enables antegrade blood flow during revascularisation, offering a potentially limitless timeframe within which to operate. The invention has the potential to enable protected deployment of further devices distal to the site of treatment due to the central passageway created by the inventions support system.

This support can in various embodiments be a shape-memory alloy, a mechanical support, an inner balloon or otherwise.

Fig. 18 illustrates a perspective cut-away view of an alternative embodiment of annular filter, 50, consisting of a filter mesh 51 folded over at 52 from a central passageway 53 and retained by shape memory alloy metal wires 54 connecting the catheter to the outer diameter of the annular filter. The shape memory alloy wires 54 are attached from a central catheter 55 to the outer diameter of the annular filter 51.

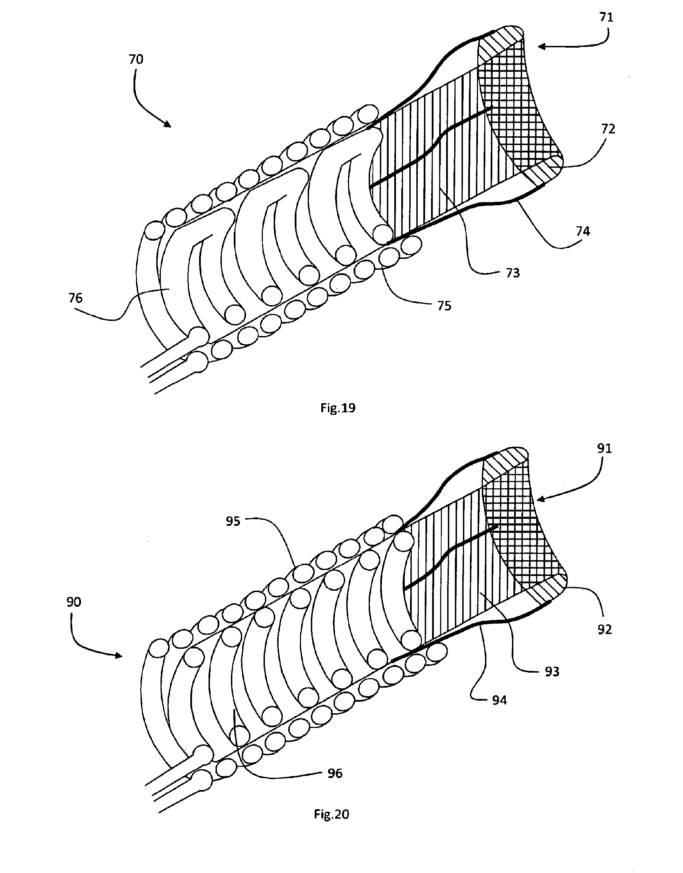

Fig. 19 illustrates a perspective cut-away view of a device 70 with a rib-cage balloon inner support structure 76, a coiled helical outer balloon 75, and a filter 71. The filter 71 has a capture region 72, a stem 73, and retainer wires 74.

Fig. 20 illustrates a two-balloon angioplasty device 90 having a filter 91 with an annular capture region 92, a central passageway stem 93, and support wires 94. There is a coiled outer balloon 95 and a coiled inner support balloon 96. This drawing illustrates how the pitch diameters can vary with respect to each other along the length of the balloons. This can be used as a mechanism to prevent the coils of each balloon slipping between one another.

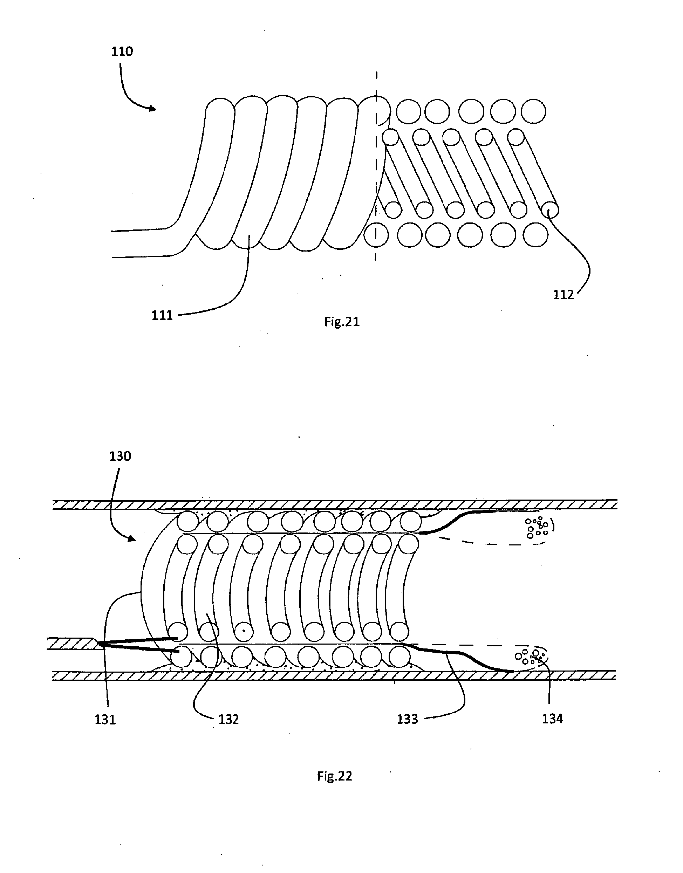

Fig 21 shows another balloon arrangement 110 for a device of the invention. There is an outer coiled balloon 111 and a coiled inner balloon 112. This embodiment demonstrates the different coil pitch angles that can be employed to provide additional support so that the balloons remain isolated and stable.

Fig. 22 illustrates a device 130 deployed in an artery. There is an outer coiled balloon 131, an inner coiled support balloon 132, filter support wires 133, and a filter capture region 134. The stem of the filter extends in-between the two balloons.

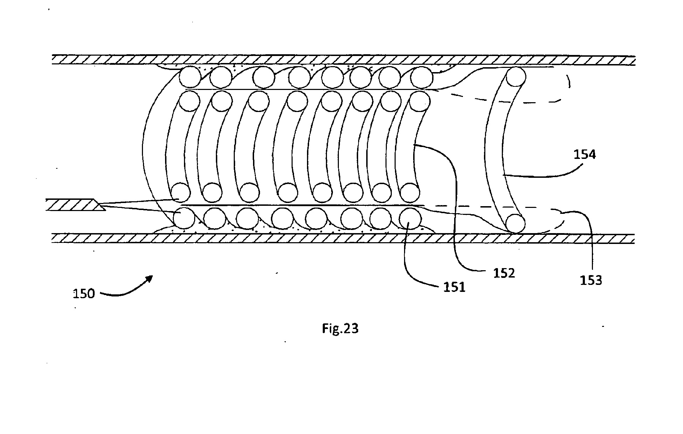

Fig. 23 illustrates a device 150 fully deployed in an artery. There is an outer coiled annular balloon 151, an inner coiled support balloon 152, and a filter 153 supported by an inflated ring 154.

Though there have been no randomised studies to date comparing CAS with and without EPD, the availability and employment of EPDs is important and has greatly reduced the risk of postprocedural complications during carotid angioplasty and stenting [5]. The device of the invention is a combination of an annular perfusion balloon and an annular perfusion filter. Prior art filters are designed to capture emboli fragments in the blood and also to permit blood to pass through the filter to distal arteries and the brain. Therefore the design of traditional filters involves a trade off in the functionality of both key design criteria through the design of the filter pore size and distribution. Larger pores increase filter permeabilty but could also permit emboli to pass through the filter which can be detrimental. However, even a filter that adheres to these criteria begins to fail as it collects emboli because the filters pores become blocked with captured emoboli which further restricts the flow of blood to the brain which can result in ischemic events. The invention provides a perfusion chamber through both the balloon and filter so that emboli-free blood can transverse, which is in contrast to traditional filters where the deposition of emboli coincides with a reduction in blood flow and an increase in the pressure drop in the vessel. The device of the invention prevents this from happening and can be designed with small pore size, to capture all potentionally detremental emboli, and have a constant supply of blood to the brain.

It will be appreciated that the invention avoids the major disadvantage with prior balloon catheters which is that in order to relieve a blockage in an artery it must first create one. When a typical prior device is inflated it blocks the whole artery and blood cannot flow during this time, particularly in patients with poor collateral blood flow.

In the invention the balloon inflates and expands the inner arterial wall with lesion, and the filter captures emboli released during the carotid angioplasty procedure. The filter extends fully between the perfusion balloon and the vessel wall, so that all dislodged emboli are captured. There is a channel that allows blood to flow to tissues distal to the blockage, maintaining antegrade flow. This may prevent the onset of an ischemic stroke or other perioperative neurological events. Due to there being a channel maintaining antegrade flow, the device would add a limitless time frame within which a surgeon can operate. This is advantageous in limiting the impact of the carotid angioplasty procedure on the host tissue and improving the health and state of the artery wall and the carotid sinus post procedure.

The device can be located at the site of the stenosis, encapsulating the plaque comprehensively, while also allowing antegrade blood flow at the site of the lesion. There is filter coverage of the entire plaque blockage during balloon pre-dilation, dilation and stent deployment so all factors which cause the plaque to be disturbed and generate emboli are protected by a safe and effective filter system.

In summary, the invention solves several problems of conventional treatment:
- Blood flow restrictions:
   Complete balloon occlusion of the vessel for extended periods of time lead to perioperative complications for the patient due to the reduced blood supply to cranial tissue
   Filters get "clogged" with embolic fragments further preventing blood flow and increasing the pressure drop across the devices lessening blood supply to distal cranial tissues
   Following the placement of a standard balloon and filter device, the pressure drop results in a transient episode of hypotension and further associated patient complications

Artery wall implications:
Controlled inflation of the device reduces the strain rate incurred by the atherosclerotic lesion, reducing the potential of emboli.
Gradual strain of the arterial wall reduces the likelihood of elastic recoil.
Gradual stretch of the carotid sinus minimizes the likelihood of haemodynamics instabilities such as post-surgery hypotension.

The invention is not limited to the embodiments described but may be varied in construction and detail. For example, inflation may be with gas or a liquid medium, depending on the application. Also, the invention can be employed as either a stent delivery system whereby a stent can be placed on the outer balloon or a self expanding stent can be deployed after the balloon is expanded, or an endograft delivery system whereby the balloon ensures endograft expansion, placement and fixation. In various embodiments the filter support wires or filter support balloon and filter net are bonded or welded to the inner surface of the outer balloon.

### References

[1] Abou-Chebl A., Gupta R., Bajzer C.T., (2004). Consequences of hemodynamic instability after carotid artery stenting. J Am Coll Cardiol. 43, 1:A20-A21
[2] Mangin L.; Medigue C.; Merle J-C.; Macquin-Mavier I.; Duvaldestin P.; Monti A.; Becquemin J-P. (2003) Cardiac autonomic control during balloon carotid angioplasty and stenting. Canadian Journal of Physiology and Pharmacology, Volume 81, Number 10, pp. 944-951
[3] Gupta, R., Horowitz, M., Jovin, T. G., (2005). Haemodynamic instability after carotid artery angioplasty and stent placement: a review of the literature. Neurosurg Focus 18 (1): E6
[4] Dangas G., Laird J.R., Satler L.F., Mehran R., Mintz G.S., Larrain G., Lansky A.J., Gruberg L., Parsons EM, Laureno R (2000). Postprocedural hypotension after carotid artery stent placement: predictors and short- and long-term clinical outcomes. Radiology, 215: 677-683
[5] Kastrup, A., Nägele, T., Gröschel, K., Schmidt, F., Vogler, E., Schulz, J., Ernemann, U., (2006). Incidence of New Brain Lesions after Carotid Stenting with and without Cerebral Protection. Stroke, 37: 2312-2316.

## Claims

1. A vascular device (1) comprising:
a perfusion balloon (3) having, when inflated, a through-hole for blood flow, and
a distal filter (4) for trapping emboli, and being configured to, when deployed extend distally from the perfusion balloon, **characterized in that**
the filter (4, 40) comprises a filtering portion which is configured to filter an annular cross-sectional area extending from a vessel wall while leaving an un-filtered central passageway (41).

2. A device as claimed in claim 1, wherein the filtering portion (4) is configured to, when deployed, extend in a direction which is at least partly radial, and then in a direction which is at least partly proximal.

3. A device as claimed in claims 1 or 2, wherein the filtering portion (42) is substantially frusto-conical in shape when deployed; and the filter (40) has a proximal stem(41) which is attached to the perfusion balloon or a support for the perfusion balloon.

4. A device as claimed in any preceding claim, wherein the filtering portion has an asymmetrical edge (46) for improved retrieval to enable improved device deliverability and extraction.

5. A device as claimed in any preceding claim, wherein the filter comprises a retainer (5) adapted to retain an end of the filtering portion in an in-use position abutting a vessel wall.

6. A device as claimed in claim 5, wherein the retainer comprises members (5) extending from the perfusion balloon or a support for the perfusion balloon; and wherein the retainer is adapted to apply a radially outward bias to urge the filter to abut a vessel wall.

7. A device as claimed in claim 6, wherein the retainer comprises cantilevered members (5).

8. A device as claimed in claim 5, wherein the retainer (8) is ring-shaped having a configuration conforming to a vessel perimeter.

9. A device as claimed in claim 8, wherein the retainer (8) is inflatable.

10. A device as claimed in any preceding claim, further comprising an internal support (2) for the perfusion balloon, the support being adapted to allow gradual and controlled inflation of the perfusion balloon.

11. A device as claimed in claim 10, wherein the support (12, 22) comprises a scaffold structure which may comprise a shape memory material.

12. A device as claimed in any of claim 11, wherein the support comprises at least one element (22) in the form of opposed arches.

13. A device as claimed in any of claims 10 to 12, wherein the support comprises an element (12) in the form of a coil.

14. A device as claimed in any of claims 10 to 13, wherein the support comprises an inner balloon (2, 76, 96, 112, 132) adapted to fit within the perfusion balloon (3, 75, 95, 111, 131).

15. A device as claimed in claim 14, wherein the inner and perfusion balloons (111, 112) are coiled, the coil pitches being in or out of phase.

16. A device as claimed in any preceding claim, wherein the device is adapted to support a stent around the perfusion balloon and a mechanism for delivery of the stent by inflation of the perfusion balloon.

## Patentansprüche

1. Vaskulärvorrichtung (1), die Folgendes umfasst:
einen Perfusionsballon (3) mit, wenn aufgeblasen, einem Durchgangsloch für den Blutfluss, und
einen distalen Filter (4) zum Abfangen von Embolismen, der dazu ausgebildet ist, sich, wenn in Einsatzstellung, distal von dem Perfusionsballon zu erstrecken, **dadurch gekennzeichnet, dass**
der Filter (4, 40) einen Filtrationsabschnitt umfasst, der dazu ausgebildet ist, einen ringartigen Querschnittsbereich zu filtern, der sich von einer Gefäßwand erstreckt und dabei einen ungefilterten mittleren Durchgang (41) lässt.

2. Vorrichtung nach Anspruch 1, wobei der Filtrationsabschnitt (4) dazu ausgebildet ist, sich, wenn in Einsatzstellung, in einer Richtung, die mindestens teilweise radial ist und dann in einer Richtung, die mindestens teilweise proximal ist, zu erstrecken.

3. Vorrichtung nach Anspruch 1 oder 2, wobei der Filtrationsabschnitt (42), wenn in der Einsatzstellung, im Wesentlichen kegelstumpfförmig ist; und der Filter (40) einen proximalen Schaft (41) aufweist, der an dem Perfusiansballan oder einer Halterung für den Perfusionsballon angebracht ist.

4. Vorrichtung nach einem der vorangehenden Ansprüche, wobei der Filtrationsabschnitt einen asymmetrischen Rand (46) zum besseren Zurückholen aufweist, um bessere Verabreichbarkeit und Entnahme der Vorrichtung zu ermöglichen.

5. Vorrichtung nach einem der vorangehenden Ansprüche, wobei der Filter einen Halter (5) umfasst, der dazu angepasst ist, ein Ende des Filtrationsabschnitts in einer Gebrauchsstellung an einer Gefäßwand anliegend zu halten.

6. Vorrichtung nach Anspruch 5, wobei der Halter Glieder (5) umfasst, die sich von dem Perfusionsballon oder einer Halterung für den Perfusionsballon erstrecken; und wobei der Halter dazu angepasst ist, eine Vorspannung radial nach außen aufzubringen, um den Filter dazu zu drängen, an einer Gefäßwand anzuliegen.

7. Vorrichtung nach Anspruch 6, wobei der Halter Kragglieder (5) umfasst.

8. Vorrichtung nach Anspruch 5, wobei der Halter (8) ringförmig ist und eine einem Gefäßumfang entsprechende Ausbildung aufweist.

9. Vorrichtung nach Anspruch 8, wobei der Halter (8) aufblasbar ist.

10. Vorrichtung nach einem der vorangehenden Ansprüche, weiter umfassend eine innere Halterung (2) für den Perfusionsballon, wobei die Halterung dazu angepasst ist, das allmähliche und kontrollierte Aufblasen des Perfusionsballons zuzulassen.

11. Vorrichtung nach Anspruch 10, wobei die Halterung (12, 22) eine Gerüststruktur umfasst, die ein Formgedächtnismaterial umfassen kann.

12. Vorrichtung nach Anspruch 11, wobei die Halterung mindestens ein Element (22) in Form von einander gegenüberliegenden Bögen umfasst.

13. Vorrichtung nach einem der Ansprüche 10 bis 12, wobei die Halterung ein Element (12) in Form einer Spirale aufweist.

14. Vorrichtung nach einem der Ansprüche 10 bis 13, wobei die Halterung einen inneren Ballon (2, 76, 96, 112, 132) umfasst, der dazu angepasst ist, in den Perfusionsballon (3, 75, 95, 111, 131) zu passen.

15. Vorrichtung nach Anspruch 14, wobei der innere und der Perfusionsballon (111, 112) spiralförmig sind und die Spiralenteilungen phasengleich oder phasenverschoben sind.

16. Vorrichtung nach einem der vorangehenden Ansprüche, wobei die Vorrichtung dazu angepasst ist, einen Stent um den Perfusionsballon und einen Mechanismus zum Verabreichen des Stents durch Aufblasen des Perfusionsballons zu halten.

## Revendications

1. Dispositif vasculaire (1) comportant :
un ballonnet de perfusion (3) ayant, quand il est gonflé, un trou traversant pour le flux sanguin, et
un filtre distal (4) destiné à piéger des emboles, et étant configuré pour, quand il est déployé, s'étendre de manière distale depuis le ballonnet de perfusion, **caractérisé en ce que**
le filtre (4, 40) comporte une partie de filtration qui est configurée pour filtrer une section transversale annulaire s'étendant depuis une paroi vasculaire tout en laissant une voie de passage centrale non filtrée (41).

2. Dispositif selon la revendication 1, dans lequel la partie de filtration (4) est configurée pour, quand elle est déployée, s'étendre dans une direction qui est au moins partiellement radiale, et ensuite dans une direction qui est au moins partiellement proximale.

3. Dispositif selon la revendication 1 ou la revendication 2, dans lequel la partie de filtration (42) est de forme sensiblement tronconique quand elle est déployée ; et le filtre (40) a une tige proximale (41) qui est attachée au ballonnet de perfusion ou à un support pour le ballonnet de perfusion.

4. Dispositif selon l'une quelconque des revendications précédentes, dans lequel la partie de filtration a un bord asymétrique (46) à des fins de retrait amélioré en vue de permettre une amélioration de l'acheminement et de l'extraction du dispositif.

5. Dispositif selon l'une quelconque des revendications précédentes, dans lequel le filtre comporte un dispositif de retenue (5) adapté à des fins de retenue d'une extrémité de la partie de filtration dans une position d'utilisation venant prendre appui sur une paroi vasculaire.

6. Dispositif selon la revendication 5, dans lequel le dispositif de retenue comporte des éléments (5) s'étendant depuis le ballonnet de perfusion ou un support pour le ballonnet de perfusion ; et dans lequel le dispositif de retenue est adapté pour exercer une sollicitation allant vers l'extérieur dans le sens radial pour solliciter le filtre à venir prendre appui sur une paroi vasculaire.

7. Dispositif selon la revendication 6, dans lequel le dispositif de retenue comporte des éléments en porte-à-faux (5).

8. Dispositif selon la revendication 5, dans lequel le dispositif de retenue (8) est en forme de bague ayant une configuration se conformant à un périmètre de vaisseau.

9. Dispositif selon la revendication 8, dans lequel le dispositif de retenue (8) est gonflable.

10. Dispositif selon l'une quelconque des revendications précédentes, comportant par ailleurs un support interne (2) pour le ballonnet de perfusion, le support étant adapté pour permettre un gonflage progressif et contrôlé du ballonnet de perfusion.

11. Dispositif selon la revendication 10, dans lequel le support (12, 22) comporte une structure en échafaudage qui peut comporter un matériau à mémoire de forme.

12. Dispositif selon la revendication 11, dans lequel le support comporte au moins un élément (22) ayant la forme d'arches opposées.

13. Dispositif selon l'une quelconque des revendications 10 à 12, dans lequel le support comporte un élément (12) ayant la forme d'une spirale.

14. Dispositif selon l'une quelconque des revendications 10 à 13, dans lequel le support comporte un ballonnet intérieur (2, 76, 96, 112, 132) adapté à des fins d'insertion à l'intérieur du ballonnet de perfusion (3, 75, 95, 111, 131).

15. Dispositif selon la revendication 14, dans lequel les ballonnets intérieur et de perfusion (111, 112) sont enroulés en spirale, le pas de spirale étant en ou hors de phase.

16. Dispositif selon l'une quelconque des revendications précédentes, dans lequel le dispositif est adapté à des fins de support d'une endoprothèse vasculaire autour du ballonnet de perfusion et d'un mécanisme pour l'acheminement de l'endoprothése vasculaire par gonflage du ballonnet de perfusion.
